# EUROPEAN PATENT SPECIFICATION

(11) **EP 1 664 032 B1**
(45) Date of publication and mention of the grant of the patent: **05.11.2008**
(21) Application number: 04781203.7
(22) Date of filing: 16.08.2004
(51) Int. Cl.: C07D 403/12, A61K 31/4184, A61K 31/4155, A61P 35/00

(54) **N-SUBSTITUTED PYRAZOLYL-AMIDYL-BENZIMIDAZOLYL C-KIT INHIBITORS**
N-SUBSTITUIERTE PYRAZOLYL-AMIDYL-BENZIMIDAZOLYL-C-KIT-INHIBITOREN
INHIBITEURS DE C-KIT PYRAZOLYLE-AMIDYLE-BENZIMIDAZOLYLE N-SUBSTITUES

(30) Priority: 21.08.2003 US 496766 P
(43) Date of publication of application: 07.06.2006
(73) Proprietor: OSI Pharmaceuticals, Inc., Melville, NY 11747 (US)
(72) Inventor: BOLGER, Joshua, Farmingdale, NY 11735 (US); CASTELHANO, Arlindo, L., Farmingdale, NY 11735 (US); CREW, Andrew, Phillip, Farmingdale, NY 11735 (US); LAUFER, Radoslaw, Farmingdale, NY 11735 (US); LI, An-Hu, Farmingdale, NY 11735 (US); SAMBROOK SMITH, Colin Peter, Oxford OX4 6LT (GB); SUN, Yingchuan, Waltham, MA 02451 (US)
(74) Representative: Blakey, Alison Jane
(86) International application number: PCT/US2004/026481
(87) International publication number: WO 2005/021537

(56) References cited:
- WO-A-02/072090
- US-B1- 6 218 388
- US-B1- 6 348 032

## Description

### BACKGROUND OF THE INVENTION

The present invention is directed to N-substituted pyrazolyl-amidyl-benzimidazolyl compounds. In particular, the present invention is directed to N-substituted pyrazolyl-amidyl-benzimidazolyl compounds that are inhibitors of the c-Kit proto-oncogene (also known as KIT, CD-117, stem cell factor receptor, mast cell growth factor receptor).

The c-Kit proto-oncogene is believed to be important in embryogenesis, melanogenesis, hematopoiesis, and the pathogenesis of mastocytosis, gastrointestinal tumors, and other solid tumors, as well as certain leukemias, including AML Accordingly, it would be desirable to develop novel compounds that are inhibitors of the c-Kit receptor.

Many of the current treatment regimes for hyperproliferative disorders (cancer) utilize compounds that inhibit DNA synthesis. Such compounds' mechanism of operation is to be toxic to cells, particularly to rapidly dividing tumor cells. Thus, their broad toxicity can be a problem to the subject patient. However, other approaches to anti-cancer agents that act other than by the inhibition of DNA synthesis have been explored to try to enhance the selectivity of the anti-cancer action and thereby reduce adverse side-effects.

It is known that a cell may become cancerous by virtue of the transformation of a portion of its DNA into an oncogene (i.e. a gene which, on activation, leads to the formation of malignant tumor cells). Many oncogenes encode proteins that are aberrant protein-tyrosine kinases capable of causing cell transformation. By a different route, the overexpression of a normal proto-oncogenic tyrosine kinase can also result in proliferative disorders, sometimes resulting in a malignant phenotype. Alternatively, co-expression of a receptor tyrosine kinase and its cognate ligand within the same cell type may also lead to malignant transformation.

Receptor tyrosine kinases are large enzymes which span the cell membrane and possess i) an extracellular binding domain for growth factors such as KIT ligand (also known as stem cell factor (SCF), Steel factor (SLF) or mast cell growth factor (MGF)), ii) a transmembrane domain, and iii) an intracellular portion which functions as a kinase to phosphorylate specific tyrosine residues in proteins. Binding of KIT ligand to KIT tyrosine kinase results in receptor homodimerization, the activation of KIT tyrosine kinase activity, and the subsequent phosphorylation of a variety of protein substrates, many of which are effectors of intracellular signal transduction, These events can lead to enhanced cell proliferation or promote enhanced cell survival. With some receptor kinases, receptor heterodimerization can also occur.

It is known that such kinases are frequently aberrantly expressed in common human cancers such as breast cancer, head and neck cancers, gastrointestinal cancer such as colon, rectal or stomach cancer, leukemia, and ovarian, bronchial, lung or pancreatic cancer. KIT kinase expression has been documented in a wide variety of human malignancies such as mastocytosis/ mast cell leukemia, gastrointestitial stromal tumors (GIST), small cell lung carcinoma (SCLC), sinonasal natural killer/T-cell lymphoma, testicular cancer (seminoma), thyroid carcinoma, malignant melanoma, ovarian carcinoma, adenoid cystic carcinoma, acute myelogenous leukemia (AML), breast carcinoma, pediatric T-cell acute lymphoblastic leukemia, angiosarcoma, anaplastic large cell lymphoma, endometrial carcinoma, and prostate carcinoma. The kinase activity of KIT has been implicated in the pathophysiology of several of these - and additional tumors - including breast carcinoma, SCLC, GIST, germ cell tumors, mast cell leukemia, neuroblastoma, AML, melanoma and ovarian carcinoma.

Several mechanisms of KIT activation in tumor cells have been reported, including activating mutations, autocrine and paracrine activation of the receptor kinase by its ligand, loss of protein-tyrosine phosphatase activity, and cross activation by other kinases. The transforming mechanisms initiated by the activating mutations are thought to include dimer formation and increased intrinsic activity of the kinase domain, both of which result in constitutive ligand-independent kinase activation, and possibly altered substrate specificity. More than thirty activating mutations of the Kit protein have been associated with highly malignant tumors in humans.

Accordingly, it has been recognized that inhibitors of receptor tyrosine kinases are useful as selective inhibitors of the growth of mammalian cancer cells. For example, Gleevec^{™} (also known as imatinib mesylate, or STI571), a 2-phenylpyrimidine tyrosine kinase inhibitor that inhibits the kinase activity of the BCR-ABL fusion gene product, was recently approved by the U.S. Food and Drug Administration for the treatment of CML. Gleevec^{™}, in addition to inhibiting BCR-ABL kinase, also inhibits the KIT kinase and PDGF receptor kinase, although it is not effective against all mutant isoforms of the KIT kinase. Kit ligand-stimulated growth of MO7e human leukemia cells is inhibited by Gleevec^{™}, which also induces apoptosis under these conditions. By contrast, GM-CSF stimulated growth of MO7e human leukemia cells is not affected by Gleevec^{™}. Further, in recent clinical studies using Gleevec^{™} to treat patients with GIST, a disease in which KIT kinase is involved in transformation of the cells, many of the patients showed marked improvement.

These studies demonstrate how KIT kinase inhibitors can treat tumors whose growth is dependent on KIT kinase activity. Other kinase inhibitors show even greater kinase selectivity. For example, the 4-anilinoquinazoline compound Tarceva® inhibits only EGF receptor kinase with high potency, although it can inhibit the signal transduction of other receptor kinases, probably by virtue of the fact that these receptors heterodimerize with EGF receptor.

Although anti-cancer compounds such as those described above make a significant contribution to the art, there is a continuing need for improved anti-cancer pharmaceuticals, and it would be desirable to develop new compounds with better selectivity or potency, or with reduced toxicity or side effects.

U.S. Patent Nos. 5,990,146 and 6,218,388 describe benzimidazoles for inhibiting protein tyrosine kinase mediated cellular proliferation. U.S. Patent No. 6,348,032 describes method of inhibiting neoplastic cells with benzimidazole derivatives. International Patent Publication No. WO 01/21634 describes benzimidazole derivatives and combinatorial libraries thereof. International Patent Publication No. WO 01/57020 describes indole and benzimidazole inhibitors of factor Xa. International Patent Publication No. WO 00/15222 describes fused pyridine inhibitors of cGMP phosphodiesterase. International Patent Publication No. WO 01/12600 describes inhibitors of Factor Xa. International Patent Publication No. WO 97/12613 describes method for treating and preventing inflammation and atherosclerosis.

U.S. Patent No. 6,316,474 describes 2-benzyl and 2-heteroaryl benzimidazole NMDA/NR2b antagonists. U.S. Patent No. 6,479,508 describes viral polymerase inhibitors. U.S. Patent No. 6,444,617 describes fused-heterocycle dicarboxylic acid diamide derivatives or salts thereof, herbicide and usage thereof. U.S. Patent Nos. 6,087,380, 6,414,008, and 6,469,039 describe disubstituted bicyclic heterocycles. U.S. Patent No. 5,118,688 describes tetrahydropyridonquinolone derivatives. U.S. Patent No. 4,975,435 describes certain 1H-pyrrolo[3,4-b]quinolin-1-one-9-amino-2,3-dihydro derivatives useful for treating anxiety. U.S. Patent No. 6,548,524 describes ortho-sulfonamido bicyclic heteroaryl hydroxamic acids. U.S. Patent No. 6,348,474 describes sulfonamide compounds.

U.S. Patent Nos. 5,972,980 and 6,001,866 describe method for treating and preventing inflammation and atherosclerosis. U.S. Patent No. 5,814,651 describes catechol diethers as selective PDEIV inhibitors. U.S. Patent No. 6,329,383 describes 2-amino-5-pyrimidine acetic acid compounds. U.S. Patent No. 5,688,809 describes 5-heteroarylindole derivatives. European Patent Application No. EP 0 846 689 describes benzimidazole compounds. International Patent Publication No. WO 00/59888 describes N-benzimidazolylmethyl- and N-indolylmethyl-benzamides and their use as CRF modulators. International Patent Publication No. WO 02/069965 describes benzimidazole derivatives as therapeutic agents. International Patent Publication No. WO 02/30886 describes heterocyclic angiogenesis inhibitors. U.S. Patent No. 6,162,804 describes tyrosine kinase inhibitors. U.S. Patent No. 6,465,484 describes angiogenesis inhibitors. International Patent Publication No. WO 00/12089 describes novel angiogenesis inhibitors.

German Patent Publication No. DE 2244908 describes selectively permeable polymeric membranes. European Patent Application No. EP 0 706 795 describes catechol diether compounds as inhibitors of TNF release. International Patent Publication No. WO 02/076960 describes transition metal mediated process. International Patent Publication No. WO 02/059118 describes process for N-(oxyalkylation) of carboxamides. International Patent Publication No. WO 02/04425 describes viral polymerase inhibitors. International Patent Publication No. WO 02/083143 describes CXCR3 antagonists. International Patent Publication No. WO 01/57019 describes indolone and benzimidazolone inhibitors of factor Xa. European Patent Application No. EP 1 085 372 describes photographic material having improved color reproduction. International Patent Publication No. WO 01/14342 describes aminocarbonyl-substituted benzimidazole derivatives. International Patent Publication No. WO 00/76501 describes IL-8 receptor antagonists. International Patent Publication No. WO02/072090 describes benzimidazole derivatives as modulators of IgE and inhibitors of cellular proliferation.

Thus, it is desirable to develop compounds that exhibit Kit inhibition and are useful in oncology. Further, such compounds may be active in other kinases such as, for example, GIST, FLT3, Hematopoietic R-PTKs, PDGFR-beta or KDR to add efficacy in mast cell leukemias, small cell lung cancer (SCLC), mastocytosis, leukemias, myelodysplastic disorders, or angiogenic dependent diseases.

### SUMMARY OF THE INVENTION

Compounds represented by Formula (I): 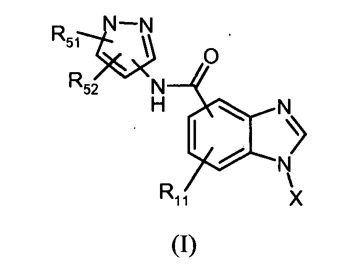 or a pharmaceutically acceptable salt or N-oxide thereof, are useful in the treatment of tumors.

### DETAILED DESCRIPTION OF THE INVENTION

The present invention is directed to a compound represented by Formula (1): 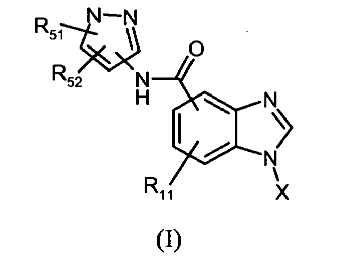 or a pharmaceutically acceptable salt or N-oxide thereof, wherein

R₁₁ is F, Cl, C₀₋₈alkyl, C₀₋₈alkoxy, or -N(C₀₋₈alkyl)(C₀₋₈alkyl);

R₅₁ is phenyl, optionally substituted with 1-5 independent halogen, -NR₃₄R₃₅, -NR₃₄COR₃₅, -NR₃₄C(O)OR₃₅, -NR₃₄SO₂R₃₅, -OR₃₄, -SR₃₄, -SO₂R₃₄, -SO₂NR₃₄R₃₅, -C(O)OR₃₄, -CO₂H, -CONR₃₄R₃₅, C₀₋₈alkyl, C₂₋₈alkenyl, C₂₋₈alkynyl, CN, CF₃, NO₂, oxo, carbocyclic or heterocyclyl substituents;

R₅₂ is halogen, CF₃, -CN, C₀₋₈alkyl, C₀₋₈alkoxy, -COOH, or -N(C₀₋₈alkyl)(C₀₋₈alkyl);

X is a carbocyclic or heterocyclyl group, optionally substituted with 1-4 independent F, Cl, CF₃, -CN, C₀₋₈alkyl, C₀₋₈alkoxy, -COOH, or -N(C₀₋₈alkyl)(C₀₋₈alkyl) substituents;

R₃₄ and R₃₅ are independently C₀₋₈alkyl optionally substituted with a heterocyclyl or OH substituent; -C₀₋₈alkyl-C₃₋₈cycloalkyl, CF₃, -C₀₋₈alkyl-O-C₀₋₈-alkyl, -C₀₋₈alkyl-N(C₀₋₈alkyl)(C₀₋₈alkyl), -C₀₋₈alkyl-S(O)₀₋₂-C₀₋₈alkyl; or heterocyclyl optionally substituted with C₀₋₈alkyl, a carbocyclic or substituted carbocyclic substituent.

In one aspect, the present invention is directed to a compound represented by Formula (I), or a pharmaceutically acceptable salt or N-oxide thereof, wherein X is an optionally substituted carbocyclic group, and the other variables are as described above for Formula (I).

In an embodiment of the aspect, the present invention is directed to a compound represented by Formula (I), or a pharmaceutically acceptable salt or N-oxide thereof, wherein X is optionally substituted phenyl, and the other variables are as described above for Formula (I).

In a second aspect, the present invention is directed to a compound represented by Formula (I), or a pharmaceutically acceptable salt or N-oxide thereof, wherein X is optionally substituted heterocyclyl, and the other variables are as described above for Formula (I).

As used herein, unless stated otherwise, "alkyl" as well as other groups having the prefix "alk" such as, for example, alkoxy, alkanyl, alkenyl, alkynyl, and the like, means carbon chains which may be linear or branched or combinations thereof. Examples of alkyl groups include methyl, ethyl, propyl, isopropyl, butyl, sec- and tert-butyl, pentyl, hexyl, heptyl and the like. "Alkenyl", "alkynyl" and other like terms include carbon chains having at least one unsaturated carbon-carbon bond.

As used herein, "C₀₋₄alkyl" is used to mean an alkyl having 0-4 carbons - that is, 0, 1, 2, 3, or 4 carbons in a straight or branched configuration. An alkyl having no carbon is hydrogen when the alkyl is a terminal group. An alkyl having no carbon is a direct bond when the alkyl is a bridging (connecting) group.

The terms "cycloalkyl", "carbocyclic ring", "cyclic", or "cyclyl" mean 3-10 membered mono or polycyclic aromatic, partially aromatic or non-aromatic ring carbocycles containing no heteroatoms, and include mono-, bi-, and tricyclic saturated carbocycles, as well as fused and bridged systems. Such fused ring systems can include one ring that is partially or fully unsaturated, such as a benzene ring, to form fused ring systems, such as benzofused carbocycles. Cycloalkyl includes such fused ring systems as spirofused ring systems. Examples of cycloalkyl and carbocyclic rings include C₃₋₈cycloalkyl such as cyclopropyl, cyclobutyl, cyclopentyl, cyclohexyl, and decahydronaphthalene, adamantane, indanyl, 1,2,3,4-tetrahydronaphthalene and the like.

The term "halogen" includes fluorine, chlorine, bromine, and iodine atoms.

The term "carbamoyl" unless specifically described otherwise means -C(O)-NH- or -NH-C(O)-.

The term "aryl" is well known to chemists. The preferred aryl groups are phenyl and naphthyl.

The term "hetaryl" is well known to chemists. The term includes 5- or 6- membered heteroaryl rings containing 1-4 heteroatoms chosen from oxygen, sulfur, and nitrogen in which oxygen and sulfur are not next to each other. Examples of such heteroaryl rings are furyl, thienyl, pyrrolyl, pyrazolyl, imidazolyl, oxazolyl, isoxazolyl, thiazolyl, isothiazolyl, triazolyl, oxadiazolyl, thiadiazolyl, tetrazolyl, pyridyl, pyridazinyl, pyrimidinyl, pyrazinyl, and triazinyl. The term "hetaryl" includes hetaryl rings with fused carbocyclic ring systems that are partially or fully unsaturated, such as a benzene ring, to form a benzofused hetaryl. For example, benzimidazole, benzoxazole, benzothiazole, benzofuran, quinoline, isoquinoline, quinoxaline, and the like.

Unless otherwise stated, the terms "heterocyclic ring", "heterocycle", "heterocyclic", and "heterocyclyl" are equivalent, and is defined as for cyclic but also contains one or more atoms chosen independently from N, O, and S and their oxides, provided such derivatives exhibit appropriate and stable valencies and excludes moieties containing O-O, S(O)ₙ-S(O)ₙ, S(O)ₙ-O bonds where n=0-2. The terms include 4-8-membered saturated rings containing one or two heteroatoms chosen from oxygen, sulfur, and nitrogen. Examples of heterocyclic rings include azetidine, oxetane, tetrahydrofuran, tetrahydropyran, oxepane, oxocane, thietane, thiazolidine, oxazolidine, oxazetidine, pyrazolidine, isoxazolidine, isothiazolidine, tetrahydrothiophene, tetrahydrothiopyran, thiepane, thiocane, azetidine, pyrrolidine, piperidine, azepane, azocane, [1,3]dioxane, oxazolidine, piperazine, homopiperazine, morpholine, thiomorpholine, and the like. Other examples of heterocyclic rings include the oxidized forms of the sulfur-containing rings. Thus, tetrahydrothiophene-1-oxide, tetrahydrothiophene- 1, I-dioxide, thiomorpholine-1-oxide, thiomorpholine-1,1-dioxide, tetrahydrothiopyran-1-oxide, tetrahydrothiopyran-1,1-dioxide, thiazolidine-1-oxide, and thiazolidine-1,1-dioxide are also considered to be heterocyclic rings. The term "heterocyclic" also includes fused ring systems and can include a carbocyclic ring that is partially or fully unsaturated, such as a benzene ring, to form benzofused heterocycles. For example, 3,4,-dihydro-1,4-benzodioxine, tetrahydroquinoline, tetrahydroisoquinoline and the like.

Compounds described herein may contain one or more asymmetric centers and may thus give rise to diastereomers and optical isomers. The present invention includes all such possible diastereomers as well as their racemic mixtures, their substantially pure resolved enantiomers, all possible geometric isomers, and pharmaceutically acceptable salts thereof. The above Formula I is shown without a definitive stereochemistry at certain positions. The present invention includes all stereoisomers of Formula I and pharmaceutically acceptable salts thereof. Further, mixtures of stereoisomers as well as isolated specific stereoisomers are also included. During the course of the synthetic procedures used to prepare such compounds, or in using racemization or epimerization procedures known to those skilled in the art, the products of such procedures can be a mixture of stereoisomers.

The invention also encompasses a pharmaceutical composition that is comprised of a compound of Formula I in combination with a pharmaceutically acceptable carrier.

Preferably, the composition is comprised of a pharmaceutically acceptable carrier and a non-toxic therapeutically effective amount of a compound of Formula I as described above (or a pharmaceutically acceptable salt or N-oxide thereof).

Moreover, within this preferred embodiment, the invention encompasses a pharmaceutical composition for the treatment of disease by the inhibition of the c-Kit kinase, which may be a wild-type or mutant form of the protein, comprising a pharmaceutically acceptable carrier and a non-toxic therapeutically effective amount of compound of Formula I as described above (or a pharmaceutically acceptable salt or N-oxide thereof).

The compounds and compositions of the present invention are effective for treating mammals such as, for example, humans.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, manganese (ic and ous), potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include ion exchange resins such as, for example, arginine, betaine, caffeine, choline, N',N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Particularly preferred are citric, hydrochloric, maleic, phosphoric, sulfuric, methanesulfonic, and tartaric acids.

The pharmaceutical compositions of the present invention comprise a compound represented by formula I (or a pharmaceutically acceptable salt or N-oxide thereof) as an active ingredient, a pharmaceutically acceptable carrier and optionally other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy.

In practice, the compounds represented by Formula I, or pharmaceutically acceptable salts or N-oxides thereof, of this invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). Thus, the pharmaceutical compositions of the present invention can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in-oil liquid emulsion. In addition to the common dosage forms set out above, the compound represented by Formula I, or a pharmaceutically acceptable salt or N-oxide thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

Thus, the pharmaceutical compositions of this invention may include a pharmaceutically acceptable carrier and a compound or a pharmaceutically acceptable salt or N-oxide of Formula I. The compounds of Formula I, or pharmaceutically acceptable salts or N-oxides thereof, can also be included in pharmaceutical compositions in combination with one or more other therapeutically active compounds.

The pharmaceutical compositions of this invention include a pharmaceutically acceptable liposomal formulation containing a compound of Formula I or a pharmaceutically acceptable salt or N-oxide thereof.

The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent or other such excipient. These excipients may be, for example, inert diluents such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example, magnesium stearate, stearic acid or talc. The tablets may be uncoated or they may be coated by known techniques to delay disintegration and absorption in the gastrointestinal tract and thereby provide a sustained action over a longer time. For example, a time delay material such as glyceryl monostearate or glyceryl distearate may be used.

In hard gelatin capsules, the active ingredient is mixed with an inert solid diluent, for example, calcium carbonate, calcium phosphate or kaolin. In soft gelatin capsules, the active ingredient is mixed with water or an oil medium, for example, peanut oil, liquid paraffin or olive oil. Molded tablets may be made by molding in a suitable machine, a mixture of the powdered compound moistened with an inert liquid diluent. Each tablet preferably contains from about 0.05mg to about sag of the active ingredient and each cachet or capsule preferably containing from about 0.05mag to about 5g of the active ingredient.

For example, a formulation intended for the oral administration to humans may contain from about 0.5mg to about 5g of active agent, compounded with an appropriate and convenient amount of carrier material, which may vary from about 5 to about 95 percent of the total composition. Unit dosage forms will generally contain between from about 1mg to about 2g of the active ingredient, typically 25mg, 50mg, 100mg, 200mg, 300mg, 400mg, 500mg, 600mg, 800mg, or 1000mg.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, utilizing a compound represented by Formula I of this invention, or a pharmaceutically acceptable salt or N-oxide thereof, via conventional processing methods. As an example, a cream or ointment is prepared by admixing hydrophilic material and water, together with about 5wt% to about 10wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound described by Formula I, or pharmaceutically acceptable salts or N-oxides thereof, may also be prepared in powder or liquid concentrate form.

Generally, dosage levels on the order of from about 0.01mg/kg to about 150mg/kg of body weight per day are useful in the treatment of the above-indicated conditions, or alternatively about 0.5mg to about 10g per patient per day. For example, breast cancer, head and neck cancers, and gastrointestinal cancer such as colon, rectal or stomach cancer may be effectively treated by the administration of from about 0.01 to 100mg of the compound per kilogram of body weight per day, or alternatively about 0.5mg to about 7g per patient per day.

Similarly, leukemia, ovarian, bronchial, lung, and pancreatic cancer may be effectively treated by the administration of from about 0.01 to 100mg of the compound per kilogram of body weight per day, or alternatively about 0.5mg to about 7g per patient per day.

Mastocytosis/ mast cell leukemia, gastrointestinal stromal tumors (GIST), small cell lung carcinoma (SCLC), sinonasal natural killer/T-cell lymphoma, testicular cancer (seminoma), thyroid carcinoma, malignant melanoma, ovarian carcinoma, adenoid cystic carcinoma, acute myelogenous leukemia (AML), breast carcinoma, pediatric T-cell acute lymphoblastic leukemia, angiosarcoma, anaplastic large cell lymphoma, endometrial carcinoma, and prostate carcinoma may be effectively treated by the administration of from about 0.01 to 100mg of the compound per kilogram of body weight per day, or alternatively about 0.5mg to about 7g per patient per day.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

The compounds of the present invention, or pharmaceutically acceptable salts or N-oxides thereof, can also be effectively administered in conjunction with other cancer therapeutic compounds. For example, cytotoxic agents and angiogenesis inhibiting agents can be advantageous co-agents with the compounds of the present invention. Accordingly, the present invention includes compositions comprising the compounds represented by Formula I, or a pharmaceutically acceptable salt or N-oxide thereof, and a cytotoxic agent or an angiogenesis-inhibiting agent. The amounts of each can be therapeutically effective alone - in which case the additive effects can overcome cancers resistant to treatment by monotherapy. The amounts of any can also be subtherapeutic - to minimize adverse effects, particularly in sensitive patients.

It is understood that the treatment of cancer depends on the type of cancer. For example, lung cancer is treated differently as a first line therapy than are colon cancer or breast cancer treated. Even within lung cancer, for example, first line therapy is different from second line therapy, which in turn is different from third line therapy. Newly diagnosed patients might be treated with cisplatinum containing regimens. Were that to fail, they move onto a second line therapy such as a taxane. Finally, if that failed, they might get a tyrosine kinase EGFR inhibitor as a third line therapy. Further, the regulatory approval process differs from country to country. Accordingly, the accepted treatment regimens can differ from country to country. Nevertheless, the compounds of the present invention, or pharmaceutically acceptable salts or *N*-oxides thereof, can be beneficially co-administered in conjunction or combination with other such cancer therapeutic compounds. Such other compounds include, for example, a variety of cytotoxic agents (alkylators, DNA topoisomerase inhibitors, antimetabolites, tubulin binders); inhibitors of angiogenesis; and different other forms of therapies including kinase inhibitors such as Tarceva®, monoclonal antibodies, and cancer vaccines. Other such compounds that can be beneficially co-administered with the compounds of the present invention include doxorubicin, vincristine, cisplatin, carboplatin, gemcitabine, and the taxanes. Thus, the compositions of the present invention include a compound according to Formula I, or a pharmaceutically acceptable salt or N-oxide thereof, and an anti-neoplastic, anti-tumor, anti-angiogenic, or chemotherapeutic agent.

The compounds of the present invention, or pharmaceutically acceptable salts or N-oxides thereof, can also be effectively administered in conjunction with other therapeutic compounds, aside from cancer therapy. For example, therapeutic agents effective to ameliorate adverse side-effects can be advantageous co-agents with the compounds of the present invention.

### I. Assay for inhibition of c-Kit in intact cells

The ability of compounds to inhibit the tyrosine kinase activity of c-Kit was determined in a cell-based ELISA assay using the H526 cell line (ATCC # CRL-5811), which was originally derived from a human small cell lung cancer. The assay determines the ability of compounds to block ligand-stimulated tyrosine phosphorylation of the wild-type c-Kit receptor protein that is endogenously expressed in H526 cells. Cells are pre-incubated with compounds at various concentrations prior to addition of stem cell factor (SCF), the ligand for the c-Kit receptor tyrosine kinase. Cell lysates are then prepared and the c-Kit protein is captured onto a c-Kit antibody-coated 96-well ELISA plate. The phosphotyrosine content of the receptor protein is then monitored by quantitation of the degree of binding of an antibody that recognizes only the phosphorylated tyrosine residues within the captured protein. The antibody used has a reporter enzyme (e.g. horseradish peroxidase, HRP) covalently attached, such that binding of antibody to phosphorylated c-Kit can be determined quantitatively by incubation with an appropriate HRP substrate.

The stock reagents used are as follows:
**Cell lysis buffer:**
   50 mM Tris-HCl, pH 7.4
   150 mM NaCl
   10% Glycerol
   1% Triton X-100
   0.5 mM EDTA
   1 µg/mL leupeptin
   1 µg/mL aprotinin
   1 mM Sodium orthovanadate
**Anti c-Kit antibody:**
   0.5µg/mL anti c-Kit Ab-3 (Lab Vision, catalog #MS289P1) in 50mM Sodium bicarbonate, pH 9.
**ELISA Assay plates:**
   ELISA assay plates are prepared by addition of 100µL of anti c-Kit antibody to each well of a 96-well Microlite-2 plate (Dynex, catalog # 7417), followed by incubation at 37°C for 2h. The wells are then washed twice with 300µL wash buffer.
**Plate wash buffer:**
   PBS containing 0.5% Tween-20 (PBST)
**Cell assay medium:**
   RPMI with 0.1% BSA
**pY20-HRP:**
   25ng/mL pY20-HRP (Calbiochem, catalog # 525320) in PBS, containing 0.5% Tween-20, 5% BSA, 1 mM Sodium orthovanadate
**HRP substrate:**
   Chemoluminescent detection reagent (Pierce, catalog # 37075)
**Assay protocol:**
   Cultures of H526 cells, growing in RPMI with 10% fetal calf serum, were collected by centrifugation, washed twice with PBS, and suspended in cell assay medium. Cells were then distributed into a V-bottom 96-well plate at 7.5 x 10⁴ cells per well in 100µL cell assay medium.

Compound dilutions were prepared from 10mM DMSO stocks by dilution in cell assay medium, the final concentration of DMSO in the assay being 0.1%. To compound incubation wells, 50µL of the test compound was added (compounds are assayed at concentrations between 0.1nM and 100µM); to positive and negative control wells, 50µL cell assay medium containing 0.1% DMSO was added. The cells were then incubated with compound at 37°C for 3h. SCF (R&D Systems, catalog #255-SC-010) was then added in order to stimulate the Kit receptor and induce its tyrosine phosphorylation. Then, 10µL of a 1.6µg/mL solution of SCF in cell assay medium was added to all wells apart from the negative control wells, and the cells were incubated for an additional 15min at 37°C. Following the addition of ice-cold PBS, the plate was centrifuged at 1000rpm for 5min, the medium removed by aspiration, and the cell pellet lysed by the addition of 120µL ice-cold cell lysis buffer per well. The plate was kept on ice for 20min and 100µL of the cell lysates from each well were then transferred to the wells of an ELISA assay plate and incubated at 4°C for 16h.

Following incubation of the cell lysates in the ELISA plate, the wells were washed 4 times with 300µL wash buffer, then 100µL of the phosphotyrosine detection antibody pY20-HRP was added to each well and the plate incubated at rt for 2h. The wells were then washed 4 times with 300µL wash buffer. Then, 50µL of the chemiluminescent HRP substrate was added to each well for luminometric quantitation of the amount of antiphosphotyrosine-HRP conjugate bound to the plate.

Comparison of the assay signals obtained in the presence of compound with those of the positive and negative controls (cells incubated in the presence or absence of SCF, with no compound added), allows the degree of inhibition of c-Kit receptor tyrosine phosphorylation to be determined over a range of compound concentrations. These inhibition values were fitted to a sigmoidal dose-response inhibition curve to determine the IC₅₀ values (i.e. the concentration of compound that inhibits SCF-induced tyrosine phosphorylation of the c-Kit protein by 50%).

The **EXAMPLES** of this invention reduced the level of SCF-induced tyrosine phosphorylation of Kit in intact H526 cells as determined in the above assay with IC₅₀ values less than 15µM. It is preferable that the IC₅₀ value be less than 1µM. It is more preferable that the IC₅₀ value be less than 100nM, even more preferable less than 30nM.

### EXPERIMENTAL

The **EXAMPLES** of the present invention were prepared according to the following procedures by the methods illustrated in the following schemes. Appropriate solvents, temperatures, pressures and other reaction conditions may be readily selected by one of ordinary skill in the art. Similarly, suitable starting materials may be commercially obtained or readily prepared by one skilled in the art.

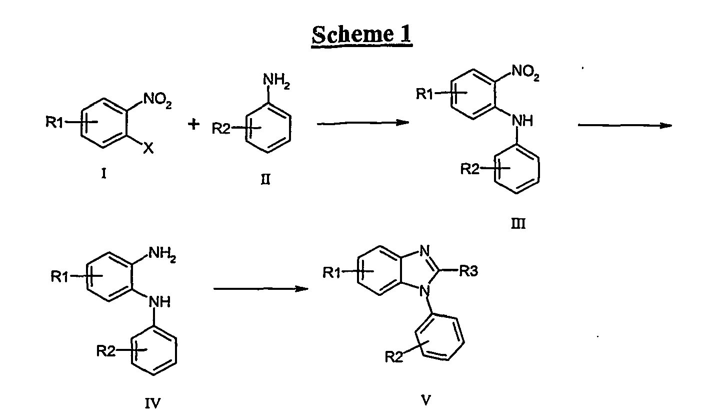

In **Scheme 1**, diarylamines (**III**) may be produced from the condensation of nitrobenzenes (I, X = F, OMS, OTs) with substituted anilines (**II**). Coupling of the anilines (**II**) may also be achieved where X = I, Br, Cl, OTf by utilisation of Pd(0) mediated Buchwald-Hartwig-type conditions or with Cu(I) catalysts and base (e.g. K₂CO₃). Reduction of **III** to give the phenylenediamines (**IV**) may be achieved using for example, hydrogen in the presence of a suitable transition metal catalyst (palladium, platinum, ruthenium, nickel), iron, zinc or tin under acidic conditions, with sodium hydrosulphite or with tin(II)chloride dihydrate. Cyclisation of **IV** to the benzimidazoles (**V**) may be achieved by reaction with a corresponding carboxylic acid, acid halide, acid anhydride or an orthoformate (e.g. (MeO)₃CH)) and an acid such as formic or *p*-toluenesulphonic acid. Under certain conditions used to reduce **III** e.g. iron powder in formic acid, conversion to the benzimidazoles **V** may be achieved in one pot. Also, by inclusion of trimethyl orthoformate into a hydrogenation mixture with **III,** allows the direct conversion to **V**.

**Scheme 2** below shows that formation ofN-arylbenzimidazoles (**V**) may also be accomplished via the process outlined, whereby N¹H benzimidazoles (**VIII**) may be arylated under Pd(0) mediated conditions as disclosed in *J. Am. Chem. Soc.,* (2000), *122*, 7600. Benzimidazoles (**VIII**) may be produced from the cyclisation of the anilides (**VII**) with acids such as, but not limited to, acetic, p-toluenesulphonic, hydrochloric, sulphuric or phosphoric acid. In turn the anilides (**VII**) can be prepared by reaction of o-phenylenediamines with acid halides or anhydrides or with carboxylic acids in the presence of appropriate coupling reagents known to those skilled in the art such as, but not limited to, EDC, DCC, HOAt, HOBt, HATU, TBTU, or CDI including solid supported versions of these solution phase reagents. Where R3 = H, compounds such as **VII** may be prepared by formylation of **VI** with alkyl formates (e.g. methyl formate). In the processes described, conversion of **VI** into **VII** may also lead to the partial or complete conversion to **VIII**.

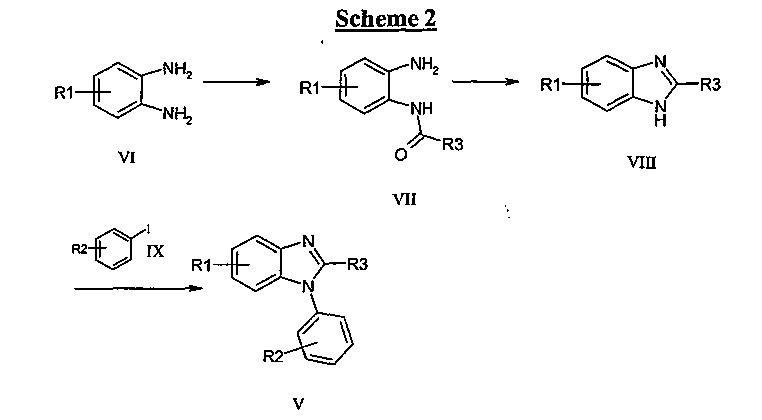

. Functionalities R1 and R2, may be included into the target molecules through appropriate choice of starting materials, e.g. of type **I**, **II**, **VI** and **IX**. Where the final functionality is not available directly through this process, or where such functionality may be compromised during the subsequent chemistry to build the final molecule, alternative functionalities may be used and subsequently transformed into the final desired functionality by methods, and at points in the sequence, readily determined by one skilled in the art. For example, a non-exhaustive list of such transformations includes the conversions: OMe→OH (BBr₃), NH₂→Cl (NaNO₂, CuCl), Br→CN (Pd₂(dba)₃, Zn(CN)₂, DPPF), Me→CO₂H (KMnO₄), CO₂H→CO₂Me (MeOH, H₂SO₄ OH→OAlkyl (Alkyl halide, base), CO₂H→CONR'R" (EDC, HOAt, DIPEA, HNR'R"), Br→CO₂Me (Pd₂(dba)₃, DPPF, CO(g), MeOH), Br→CO₂H (^{t}BuLi, CO₂), Ar-H→Ar-Br (NBS), CN→CO₂H (conc. H₂SO₄), Br→NR'R" (Pd₂(dba)₃, DPPF, HNR'R"). Representative examples of the incorporation of such functionality into target molecules are shown below in **Schemes 3** and **4**.

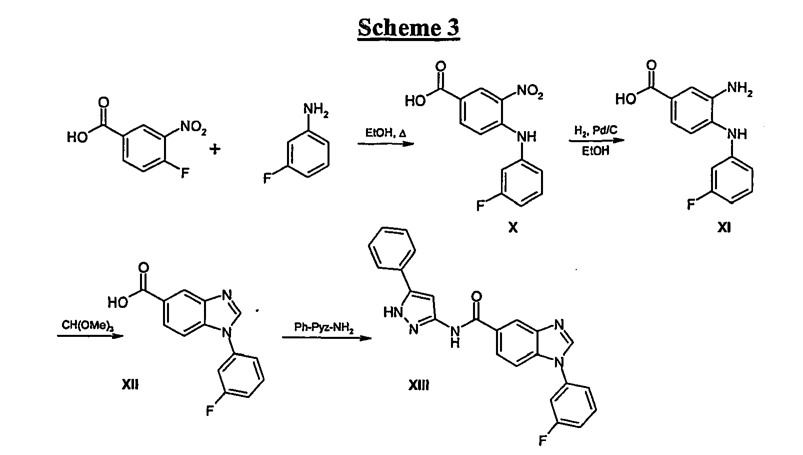

Condensation of 3-fluoroaniline with 4-fluoro-3-nitrobenzoic acid occurs through heating in ethanol to give **X** which may be reduced via catalytic hydrogenation over 10%Pd/C in ethanol to give the phenylenediamine (**XI**). Cyclisation of **XI** to the benzimidazole (**XII**) is achieved by heating with an excess of trimethylorthoformate. EDC/DMAP mediated coupling with 3-amino-5-phenylpyrazole gives target amide **XIII**. Alternatively, the benzimidazole carboxylic acid (**XII**) may be converted to its acid chloride by heating in thionyl chloride and then the isolated species reacted with the pyrazole amine in pyridine.

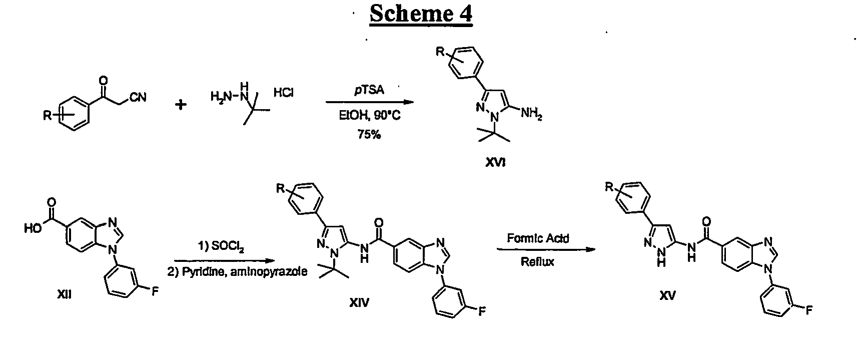

Where the aminopyrazoles were not commercially available, they could be readily prepared by various routes e.g. that shown above in **Scheme 4**. Substituted benzoylacetonitriles were cyclized in acid with *t*-butyl hydrazine hydrochloride to give 1-*t*-butyl-3-aryl-5-aminopyrazoles (**XVI**) in good yield. These could then be coupled with benzimidazole carboxylic acid chlorides derived from **XII** as described above, to afford amides **XIV**. Cleavage of the *t*-butyl group was carried out in formic acid at reflux to yield the pyrazole amides **XV**.

Definitions: EDC = ethyl dimethylaminopropylcarbodiimide hydrochloride, HOAt = 1-hydroxyazabenzotriazole, HOBt = 1-hydroxybenzotriazole, CDI = 1,1'-carbonyldiimidazole, TBTU = O-benzotriazole-N,N,N',N'-tetramethyluronium tetrafluoroborate, HATU = azabenzotriazolyl-N,N,N',N'-tetramethyluronium hexafluorophosphate, DIPEA = diisopropylethylamine, TEA = triethylamine, DMF = N,N-dimethylformamide, NMP = N-methylpyrrolidinone, DCM = dichloromethane, DMAP = 4-dimethylaminopyridine, TFA = trifluoroacetic acid, Boc = ^{t}butoxycarbonyl, Fmoc = fluorenylmethyloxycarbonyl, DMSO = dimethylsulphoxide, OMs = OSO₂Me, OTs = OSO₂-(4-Me)Ph, OTf= OSO₂CF₃, DPPF = Pd₂(dba)₃, NBS = N-bromosuccinimide, HCl (aq) = aqueous hydrochloric acid, DMA = N,N-dimethylacetamide, MeOH = methanol, EtOH = ethanol, EtOAc = ethyl acetate, DCM = dichloromethane, THF = tetrahydrofuran, HOAc = acetic acid, DMF = N,N-dimethylformamide, HPLC = high performance liquid chromatography.

### EXAMPLE B1

1-(3-Fluorophenyl)-*N*-[3(5)-phenylpyrazol-5(3)-yl]-1*H*-benzimidazole-5-carboxamide

**a**) 3-Fluoro-2-nitrobenzoic acid (4.0g, 21.6mmol) and 3-fluoroaniline (4.8g, 43.2mmol) in 15mL of ethanol were stirred at reflux under argon for 5h resulting in the formation of an orange precipitate. After 12h the heterogeneous reaction mixture was poured into 50mL of 1N HCl (aq) and diluted with 100mL of water. The solution was stirred for 20min then the precipitate filtered to yield 4-(3-fluorophenylamino)-3-nitrobenzoic acid: ¹H NMR (400 MHz, DMSO-d₆) δ 10.14 (s, 1H), 8.68 (s, 1H), 7.90 (d, *J* = 1.8 Hz, 1H), 7.26 (d, *J* = 8.8 Hz, 2H), 7.04 (d, *J* = 8.8 Hz, 2H), 6.97 (d, *J* = 6.0 Hz, 1H), 6.76 (s, 2H); MS (ES+): *m*/*z* 289 [MH⁺].

**b**) A solution of 4-(3-fluorophenylamino)-3-nitrobenzoic acid (5.57g, 20.1mmol) in THF (100mL) was charged with 10% Pd/C (500mug) and the reaction flask evacuated and subsequently charged with H₂(g) three times. The mixture was stirred vigorously for 12h after which it was filtered through diatomaceous earth and the filtrate concentrated *in vacuo* to give 3-amino-4-(3-fluorophenylamino)benzoic acid as an off-white solid: ¹H NMR (400 MHz, DMSO-d₆) δ 7.63 (s, 1H), 7.34 (d, *J* = 2.0 Hz, 1H), 7.19-7.13 (m, 2H), 7.08 (d, *J* = 8.4 Hz, 1H), 6.70 (d, *J* = 8.0 Hz, 1H), 6.60 (d, *J* = 11.6 Hz, 1H), 6.54 (t, *J* = 8.4 Hz, 1H), 5.02 (b, 1H); MS (ES+): *m*/*z* 247 [MH⁺] .

**c**) A solution of 3-amino-4-(3-fluorophenylamino)benzoic acid (4.96g, 20.1mmol) in formic acid (40mL) was charged with trimethylorthoformate (2.4mL, 22.0mmol) and heated at reflux for 3h after which time the mixture was allowed to cool to rt and stir for 12h. The reaction mixture was then poured into 150mL of H₂O and stirred for 20min yielding a precipitate which was isolated by filtration to give 1-(3-fluorophenyl)-1*H*-benzimidazole-5-carboxylic acid as an off-white solid: ¹H NMR (400 MHz, DMSO-d₆) δ 12.88 (s, 1H), 8.73 (s, 1H), 8.32 (s, 1H), 7.95 (d, *J* = 8.5 Hz, 1H), 7.75-7.66 (m, 3H), 7.59 (d, *J* = 8.0 Hz, 2H), 7.38 (t, *J* = 8.8 Hz, 1H); MS (ES+): *m*/*z* 257 [MH⁺].

**d**) A solution of 1-(3-fluorophenyl)-1*H*-benzimidazole-5-carboxylic acid (200mg, 0.78mmol) in DMF (4mL) was treated with EDC (227mg, 1. 8mmol) and DMAP (9mg, 0.07mmol) and the mixture stirred for 10min prior to the addition of 3(5)-amino-5(3)-phenylpyrazole (267mg, 1.68mmol). The mixture was stirred overnight after which time product was isolated by filtration and the cake washed with methanol (3 x 5mL) to give the title compound: ¹H NMR (400 MHz, DMSO-d₆): δ = 7.08 (s, 1 H), 7.48-7.35 (m, 4 H), 7.61 (d, *J* = 8.0 Hz, 1 H), 7.78-7.69 (m, 5 H), 8.06 (d, *J* = 8.4 Hz, 1H), 8.53 (s, 1H), 8.73 (s, 1 H), 10.93 (s, 1H), 13.21 (s, 1H). MS (ES+): *m*/*z* 398 (100) [MH⁺].

The following compounds were prepared according to the procedures used in the preparation of **EXAMPLE B1** utilizing the appropriately substituted aniline in place of 3-fluoroaniline from **Part (a)**, and substituted amine in place of 3(5)-amino-5(3)-phenylpyrazole as used in **Part (d)**.

### EXAMPLE B2

1-(3-Fluorophenyl)-*N*-[3(5)-(4-chlorophenyl)pyrazol-5(3)-yl]-1*H-*benzimidazole-5-carboxamide. MS (ES+): *m*/*z* 432 [MH⁺].

### EXAMPLE B3

1-(3-Fluorophenyl)-*N*-[3(5)-*p*-tolylpyrazol-5(3)-yl]-1*H*-benzimidazole-5-carboxamide. MS (ES+): *m*/*z* 412 [MH⁺].

### EXAMPLE B4

1-(3-Fluorophenyl)-*N*-(1-methyl-3-phenylpyrazol-5-yl)-1*H* benzimidazole-5-carboxamide. MS (ES+): *m*/*z* 412 [MH⁺].

### EXAMPLE B5

1-(4-Methoxyphenyl)-*N*-(3(5)-phenylpyrazol-5(3)-yl)-1*H*-benzimidazole-5-carboxamide. MS (ES+): *m*/*z* 409.0 [MH⁺].

### EXAMPLE B6

1-[3-Methoxy-5-(trifluoromethyl)phenyl]-*N*-(3(5)-phenylpyrazol-5(3)-yl)-1*H-*benzimidazole-5-carboxamide. MS (ES+): *m*/*z* 478.0 [MH⁺].

### EXAMPLE B7

1-(3,5-Dimethoxyphenyl)-*N*-(3(5)-phenylpyrazol-5(3)-yl)-1*H*-benzimidazole-5-carboxamide. MS (ES+): *m*/*z* 440.1 [MH⁺].

### EXAMPLE B8

1-(3-Isopropoxyphenyl)-*N*-(3(5)-phenylpyrazol-5(3)-yl)-1*H*-benzimidazole-5-carboxamide. MS (ES+): *m*/*z* 437.9 [MH⁺].

### EXAMPLE B9

. 1-(4-Bromophenyl)-*N*-(3(5)-phenylpyrazol-5(3)-yl)-1*H*-benzimidazole-5-carboxamide. MS (ES+): *m*/*z* 459.9 [MH⁺].

**General procedure for pyrazole formation:** An ethanolic solution (3mL) of 4-methoxybenzoylacetonitrile (500mg, 2.85mmol), *t*-butylhydrazine hydrochloride (391mg, 3.14mmol), and catalytic *p*-toluenesulfonic acid was stirred under argon at 90°C for 4h in a sealed tube. The reaction was concentrated and the residue partitioned between EtOAc and sat. NaHCO₃. The organic layer was washed with brine, dried with Na₂SO₄, filtered and concentrated *in vacuo.* The residue was chromatographed on silica gel with a gradient of 10-30% EtOAc in Hexanes which yielded 2-tert-butyl-5-(4-methoxyphenyl)-2H-pyrazol-3-ylamine: ¹H NMR (400 MHz, CDCl₃) δ 1.70 (s, 9H), 3.58 (br.s, 2H), 3.84 (s, 3H), 5.85 (s, 1H), 6.91 (d, *J* = 8.8 Hz, 2H), 7.69 (d, *J* = 8.8 Hz, 2H); MS (ES+): *m*/*z* 246 [MH⁺], *m*/*z* 190 [M-*^{t}*butyl].

Using this procedure the following pyrazole derivatives were also synthesized.
2-tert-Butyl-5-(4-trifluoromethylphenyl)-2H-pyrazol-3-ylamine: white solid; MS (ES+): *mlz* 228 [M-*^{t}*butyl].
2-tert-Butyl-5-*m*-tolyl-2H-pyrazol-3-ylamine: white solid; MS (ES+): *mlz* 174 [M-*^{t}*butyl].
2-tert-Butyl-5-(3-fluorophenyl)-2H-pyrazol-3-ylamine: brown; MS (ES+): *m*/*z* 177 [M-*^{t}*butyl].
2-tert-Butyl-5-(4-isopropoxyphenyl)-2H-pyrazol-3-ylamine: clear flakes; MS (ES+): *m*/*z* 218 [M-*^{t}*butyl].
2-tert-Butyl-5-(3-chlorophenyl)-2H-pyrazol-3-ylamine: brown glassy solid; MS (ES+): *m*/*z* 194 [M-*^{t}*butyl].

**Thionyl chloride procedure for coupling the aminopyrazoles (above) to the benzimidazole core:** 1-(3-Fluorophenyl)-1*H*-benzimidazole-5-carboxylic acid (100mg, 0.39mmol) was dissolved in thionyl chloride (1.5my) and stirred at 50°C for 1h. The thionyl chloride was removed by rotary evaporation and the residue dissolved in pyridine (2mL). 3-Amino-2-tert-butyl-5-(3-fluorophenyl)pyrazole in pyridine (1mL) was added and the reaction was stirred overnight. The reaction was concentrated and the residue partitioned between EtOAc and water. The organic layer was washed with water, sat. NaHCO₃, brine, dried with Na₂SO₄, filtered and concentrated *in vacuo* to a brown oil. The residue was chromatographed on silica gel with 1% MeOH in CHCl₃. The fractions were combined, concentrated and the residue crystallized from EtOH/Hexanes to give N-[2-tert-butyl-5-(3-fluorophenyl)-2H-pyrazol-3-yl] 1-(3-fluorophenyl)-1H-benzimidazole-5-carboxamide: ¹H NMR (400 MHz, CDCl₃) δ 1.79 (s, 9H), 6.84 (s, 1H), 6.97-7.01 (m, 1H), 7.25-7.39 (m, 4H), 7.57-7.65 (m, 3H), 7.72 (d, *J* = 8.4 Hz, 1H), 8.08 (dd, *J* = 8.8 Hz, *J* = 1.6 Hz, 1H), 8.16 (s, 1H), 8.20 (s, 1H), 8.46 (s, 1H); MS (ES+): *mlz* 472 [MH⁺], *mlz* 416 [M-*^{t}*butyl].

The following compounds were prepared according to the procedure described above.
N-[2-tert-butyl-5-(4-methoxyphenyl)-2H-pyrazol-3-yl] 1-(3-fluorophenyl)-1H-benzimidazole-5-carboxamide: white solid; MS (ES+): *mlz* 484 [MH⁺], *mlz* 428 [M-*^{t}*butyl].
N-[2-tert-butyl-5-(4-trifluoromethylphenyl)-2H-pyrazol-3-yl] 1-(3-fluorophenyl)-1H-benzimidazole-5-carboxamide: white solid; MS (ES+): *m*/*z* 522 [MH⁺], *m*/*z* 566 [M-*^{t}*butyl].
N-(2-tert-butyl-5-*m*-tolyl-2H-pyrazol-3-yl) 1-(3-fluorophenyl)-1H-benzimidazole-5-carboxamide: white solid; MS (ES+): *mlz* 468 [MH⁺], *mlz* 412 [M-*^{t}*butyl].
N-[2-tert-butyl-5-(4-isopropoxyphenyl)-2H-pyrazol-3-yl] 1-(3-fluorophenyl)-1H-benzimidazole-5-carboxamide: white solid; MS (ES+): *mlz* 512 [MH⁺], *m*/*z* 456 [M-*^{t}*butyl].

**General procedure for *t*-butyl cleavage:** N-(2-tert-butyl-5-*m*-tolyl-2H-pyrazol-3-yl) 1-(3-fluorophenyl)-1H-benzimidazole-5-carboxamide (110mg, 0.235mmol) was dissolved in formic acid (3mL) and the mixture stirred at reflux for 30min. The solvent was then evaporated and chased with CHCl₃ and Et₂O and the residue triturated with MeOH. The resulting precipitate was filtered off and washed with MeOH and CHCl₃ to give N-(5-*m*-tolyl-2H-pyrazol-3-yl) 1-(3-fluorophenyl)-1H-benzimidazole-5-carboxamide: ¹H NMR (400 MHz, CDCl₃) δ 2.55 (s, 3H), 7.05 (br.s, 1H), 7.18 (br.d, *J* = 8.4 Hz; 1H), 7.33-7.43 (m, 2H), 7.57 (br.d, *J* = 7.6 Hz, 1H), 7.62 (br.d, *J* = 8.0 Hz, 2H), 7.70-7.74 (m, 2H), 7.78 (d, *J* = 8.4 Hz, 1H), 8.07 (dd, *J* = 8.0 Hz, *J* = 2.0 Hz, 1H), 8.54 (s, 1H), 8.75 (s, 1H); MS (ES+): *mlz* 412 [MH⁺].
N-[5-(4-Methoxyphenyl)-2H-pyrazol-3-yl] 1-(3-fluorophenyl)-1H-benzimidazole-5-carboxamide: white solid; MS (ES+): *m*/*z* 428 [MH⁺].
N-[5-(4-Trifluoromethylphenyl)-2H-pyrazol-3-yl] 1-(3-fluorophenyl)-1H-benzimidazole-5-carboxamide: white solid; MS (ES+): *m*/*z* 466 [MH⁺].
N-[5-(3-Fluorophenyl)-2H-pyrazol-3-yl] 1-(3-fluorophenyl)-1H-benzimidazole-5-carboxamide: white solid; MS (ES+): *m*/*z* 416 [MH⁺].
N-[5-(4-Isopropoxyphenyl)-2H-pyrazol-3-yl] 1-(3-fluorophenyl)-1H-benzimidazole-5-carboxamide: white solid; MS (ES+): *m*/*z* 456 [MH⁺].

## Claims

1. A compound represented by Formula (I): or a pharmaceutically acceptable salt or N-oxide thereof, wherein
R₁₁ is F, Cl, C₀₋₈alkyl, C₀₋₈alkoxy, or N(C₀₋₈alkyl)(C₀₋₈alkyl);
R₅₁ is phenyl, optionally substituted with 1-5 independent halogen, -NR₃₄R₃₅, -NR₃₄COR₃₅, -NR₃₄C(O)OR₃₅, NR₃₄SO₂R₃₅, -OR₃₄, -SR₃₄, -SO₂R₃₄, -SO₂NR₃₄R₃₅, -C(O)OR₃₄, -CO₂H, -CONR₃₄R₃₅, C₀₋₈alkyl, C₂₋₈alkenyl, C₂₋₈alkynyl, CN, CF₃, NO₂, oxo, carbocyclic or heterocyclyl substituents;
R₅₂ is halogen, CF₃, -CN, C₀₋₈alkyl, C₀₋₈alkoxy, -COOH, or -N(C₀₋₈alkyl)(C₀₋₈alkyl);
X is a carbocyclic or heterocyclyl group, optionally substituted with 1-4 independent F, Cl, CF₃, -CN, C₀₋₈alkyl, C₀₋₈alkoxy, -COOH, or -N(C₀₋₈alkyl)(C₀₋₈alkyl) substituents;
R₃₄ and R₃₅ are independently C₀₋₈alkyl optionally substituted with a heterocyclyl or OH substituent; -C₀₋₈alkyl-C₃₋₈cycloalkyl, CF₃, -C₀₋₈alkyl-O-C₀₋₈alkyl, -C₀₋₈alkyl-N(C₀₋₈alkyl)(C₀₋₈alkyl), -C₀₋₈alkyl-S(O)₀₋₂-C₀₋₈alkyl; or heterocyclyl optionally substituted with C₀₋₈alkyl, a carbocyclic or substituted carbocyclic substituent.

2. The compound according to claim 1, or a pharmaceutically acceptable salt or N-oxide thereof, wherein X is an optionally substituted carbocyclic group.

3. The compound according to claim 2, or a pharmaceutically acceptable salt or N-oxide thereof, wherein X is optionally substituted phenyl.

4. A compound according to claim 1 consisting of
1-(3-fluorophenyl)-*N*-[3(5)-phenylpyrazol-5(3)-yl]-1*H*-benzimidazole-5-carboxamide;
1-(3-fluorophenyl)-*N*-[3(5)-(4-chlorophenyl)pyrazol-5(3)-yl]-1*H-*benzimidazole-5-carboxamide;
1-(3-fluorophenyl)-*N*-[3(5)-*p*-tolylpyrazol-5(3)-yl]-1*H*-benzimidazole-5-carboxamide;
1-(3-fluorophenyl)-*N*-(1-methyl-3-phenylpyrazol-5-yl)-1*H*-benzimidazole-5-carboxamide;
1-(4-methoxyphenyl)-*N*-(3(5)-phenylpyrazol-5(3)-yl)-1*H*-benzimidazole-5-carboxamide;
1-[3-methoxy-5-(trifluoromethyl)phenyl]-*N*-(3(5)-phenylpyrazol-5(3)-yl)-1*H-*benzimidazole-5-carboxamide;
1-(3,5-dimethoxyphenyl)-*N*-(3(5)-phenylpyrazol-5(3)-yl)-1*H*-benzimidazole-5-carboxamide;
1-(3-isopropoxyphenyl)-*N*-(3(5)-phenylpyrazol-5(3)-yl)-1*H*-benzimidazole-5-carboxamide; or
1-(4-bromophenyl)-*N*-(3(5)-phenylpyrazol-5(3)-yl)-1*H*-benzimidazole-5-carboxamide;
or a pharmaceutically acceptable salt, or *N*-oxide, thereof.

5. A composition comprising a compound according to any one of claims 1 to 4, or a pharmaceutically acceptable salt or N-oxide thereof, and a pharmaceutically acceptable carrier.

6. A composition comprising a compound according to any one of claims 1 to 4, or a pharmaceutically acceptable salt or N-oxide thereof; and an anti-neoplastic, anti-tumor, anti-angiogenic, or chemotherapeutic agent.

7. A composition comprising a compound according to any one of claims 1 to 4, or a pharmaceutically acceptable salt or N-oxide thereof, and a cytotoxic cancer therapeutic agent.

8. A composition comprising a compound according to any one of claims 1 to 4, or a pharmaceutically acceptable salt or N-oxide thereof, and an angiogenesis inhibiting cancer therapeutic agent.

9. The use of a compound according to any one of claims 1 to 4, or a pharmaceutically acceptable salt or N-oxide thereof, in the manufacture of a medicament for the treatment of a hyperproliferative disorder.

10. The use of claim 9, wherein the medicament is adapted for administration with an anti-neoplastic, anti-tumor, anti-angiogenic, or chemotherapeutic agent.

11. The use of claim 9 wherein the hyperproliferative disorder is breast cancer, head cancer, neck cancer, gastrointestinal cancer, leukemia, ovarian, bronchial, lung, or pancreatic cancer, or small cell lung or colon cancer.

12. The use of claim 9 wherein the hyperproliferative disorder is sinonasal natural killer/T-cell lymphoma, testicular cancer (seminoma), thyroid carcinoma, malignant melanoma, adenoid cystic carcinoma, angiosarcoma, anaplastic large cell lymphoma, endometrial carcinoma, or prostate carcinoma.

13. A compound according to any one of claims 1 to 4, or a pharmaceutically acceptable salt or N-oxide thereof, for use in the treatment of a hyperproliferative disorder.

14. A compound according claim 13, wherein the compound is for administration with an anti-neoplastic, anti-tumor, anti-angiogenic, or chemotherapeutic agent.

15. A compound according claim 13, wherein the hyperproliferative disorder is breast cancer, head cancer, neck cancer, gastrointestinal cancer, leukemia, ovarian, bronchial, lung, or pancreatic cancer, or small cell lung or colon cancer.

16. A compound according claim 13, wherein the hyperproliferative disorder is sinonasal natural killer/T-cell lymphoma, testicular cancer (seminoma), thyroid carcinoma, malignant melanoma, adenoid cystic carcinoma, angiosarcoma, anaplastic large cell lymphoma, endometrial carcinoma, or prostate carcinoma.

## Patentansprüche

1. Verbindungen der Formel (I): 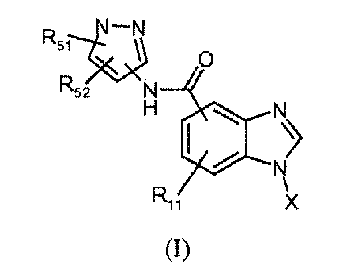 und deren pharmazeutisch unbedenkliche Salze und N-Oxide, wobei
R₁₁ für F, Cl, C₀₋₈-Alkyl, C₀₋₈-Alkoxy oder -N(C₀₋₈-Alkyl)(C₀₋₈-alkyl) steht;
R₅₁ für Phenyl, gegebenenfalls substituiert durch 1-5 unabhängige Halogen-, -NR₃₄R₃₅-, -NR₃₄COR₃₅-, -NR₃₄C(O)OR₃₅-, -NR₃₄SO₂R₃₅-, -OR₃₄-, -SR₃₄-, -SO₂R₃₄-, -SO₂NR₃₄R₃₅-, -C(O)OR₃₄-, -CO₂H-, -CONR₃₄R₃₅-, C₁₋₈-Alkyl, C₂₋₈-Alkenyl-, C₂₋₈-Alkinyl-, CN-, CF₃-, NO₂-, Oxosubstituenten, carbocyclische oder heterocyclische Substituenten, steht;
R₅₂ für Halogen, CF₃, -CN, C₀₋₈-Alkyl, C₀₋₈-Alkoxy, -COOH oder -N(C₀₋₈-Alkyl)(C₀₋₈-alkyl) steht;
X für eine carbocyclische oder heterocyclische Gruppe, gegebenenfalls substituiert durch 1-4 unabhängige F-, Cl-, CF₃-, -CN-, C₀₋₈-Alkyl-, C₀₋₈-Alkoxy-, -COOH- oder -N(C₀₋₈-Alkyl) (C₀₋₈-alkyl)-Substituenten, steht;
R₃₄ und R₃₅ unabhängig voneinander für C₀₋₈-Alkyl, gegebenenfalls substituiert durch einen Heterocyclyl- oder OH-Substituenten; -C₀₋₈-Alkyl-C₃₋₈-cycloalkyl, CF₃, -C₀₋₈-Alkyl-O-C₀₋₈-alkyl, -C₀₋₈-Alkyl-N(C₀₋₈-alkyl)(C₀₋₈-alkyl), -C₀₋₈-Alkyl-S(O)₀₋₂-C₀₋₈-alkyl; oder Heterocyclyl, gegebenenfalls substituiert durch C₀₋₈-Alkyl, einen carbocyclischen oder einen substituierten carbocyclischen Substituenten, stehen.

2. Verbindungen nach Anspruch 1 und deren pharmazeutisch unbedenkliche Salze und N-Oxide, wobei X für eine gegebenenfalls substituierte carbocyclische Gruppe steht.

3. Verbindungen nach Anspruch 2 und deren pharmazeutisch unbedenkliche Salze und N-Oxide, wobei X für ein gegebenenfalls substituiertes Phenyl steht.

4. Verbindungen nach Anspruch 1, ausgewählt aus
1-(3-Fluorphenyl)-*N*-[3(5)-phenylpyrazol-5(3)-yl]-1*H*-benzimidazol-5-carbonsäureamid;
1-(3-Fluorphenyl)-*N*-[3(5)-(4-chlorphenyl)pyrazol-5(3)-yl]-1*H*-benzimidazol-5-carbonsäureamid;
1-(3-Fluorphenyl)-*N*-[3(5)-p-tolylpyrazol-5(3)-yl]-1*H*-benzimidazol-5-carbonsäureamid;
1-(3-Fluorphenyl)-*N*-(1-methyl-3-phenylpyrazol-5-yl)-1*H*-benzimidazol-5-carbonsäureamid;
1-(4-Methoxyphenyl)-*N*-(3(5)-phenylpyrazol-5(3)-yl)-1*H*-benzimidazol-5-carbonsäureamid;
1-[3-Methoxy-5-(trifluormethyl)phenyl]-*N*-(3(5)-phenylpyrazol-5(3)-yl)-1*H*-benzimidazol-5-carbonsäureamid;
1-(3,5-Dimethoxyphenyl]-*N*-(3(5)-phenylpyrazol-5(3)-yl)-1*H*-benzimidazol-5-carbonsäureamid;
1-(3-Isopropoxyphenyl)-*N*-(3(5)-phenylpyrazol-5(3)-yl)-1*H*-benzimidazol-5-carbonsäureamid; oder
1-(4-Bromphenyl)-*N*-(3(5)-phenylpyrazol-5(3)-yl)-1*H*-benzimidazol-5-carbonsäureamid;
und deren pharmazeutisch unbedenklichen Salzen und *N*-Oxiden.

5. Zusammensetzung, enthaltend eine Verbindung nach einem der Ansprüche 1 bis 4 oder ein pharmazeutisch unbedenkliches Salz oder N-Oxid davon und einen pharmazeutisch unbedenklichen Träger.

6. Zusammensetzung, enthaltend eine Verbindung nach einem der Ansprüche 1 bis 4 oder ein pharmazeutisch unbedenkliches Salz oder N-Oxid davon und ein antineoplastisches Mittel, ein Antitumormittel, ein antiangiogenes Mittel oder ein chemotherapeutisches Mittel.

7. Zusammensetzung, enthaltend eine Verbindung nach einem der Ansprüche 1 bis 4 oder ein pharmazeutisch unbedenkliches Salz oder N-Oxid davon und ein cytotoxisches, krebstherapeutisches Mittel.

8. Zusammensetzung, enthaltend eine Verbindung nach einem der Ansprüche 1 bis 4 oder ein pharmazeutisch unbedenkliches Salz oder N-Oxid davon und ein angiogenesehemmendes, krebstherapeutisches Mittel.

9. Verwendung einer Verbindung nach einem der Ansprüche 1 bis 4 oder eines pharmazeutisch unbedenklichen Salzes oder N-Oxids davon bei der Herstellung eines Medikaments zur Behandlung einer hyperproliferativen Erkrankung.

10. Verwendung nach Anspruch 9, wobei das Medikament für die Verabreichung mit einem antineoplastischen Mittel, einem Antitumormittel, einem antiangiogenen Mittel oder einem chemotherapeutischen Mittel ausgelegt ist.

11. Verwendung nach Anspruch 9, wobei es sich bei der hyperproliferativen Erkrankung um Brustkrebs, Kopfkrebs, Halskrebs, Magen-Darm-Krebs, Leukämie, Eierstockkrebs, Bronchialkrebs, Lungenkrebs oder Bauchspeicheldrüsenkrebs oder kleinzelligen Lungenkrebs oder Dickdarmkrebs handelt.

12. Verwendung nach Anspruch 9, wobei es sich bei der hyperproliferativen Erkrankung um sinonasales Natural-Killer-/T-Zellen-Lymphom, Hodenkrebs (Seminom), Schilddrüsenkarzinom, malignes Melanom, cystisches Adenokarzinom, Angiosarkom, großzelliganaplastisches Lymphom, Endometriumkarzinom oder Prostatakarzinom handelt.

13. Verbindung nach einem der Ansprüche 1 bis 4 oder eines pharmazeutisch unbedenklichen Salzes oder N-Oxids davon zur Verwendung bei der Behandlung einer hyperproliferativen Erkrankung.

14. Verbindung nach Anspruch 13, wobei die Verbindung für die Verabreichung mit einem antineoplastischen Mittel, einem Antitumormittel, einem antiangiogenen Mittel oder einem chemotherapeutischen Mittel vorgesehen ist.

15. Verbindung nach Anspruch 13, wobei es sich bei der hyperproliferativen Erkrankung um Brustkrebs, Kopfkrebs, Halskrebs, Magen-Darm-Krebs, Leukämie, Eierstockkrebs, Bronchialkrebs, Lungenkrebs oder Bauchspeicheldrüsenkrebs oder kleinzelligen Lungenkrebs oder Dickdarmkrebs handelt.

16. Verbindung nach Anspruch 13, wobei es sich bei der hyperproliferativen Erkrankung um sinonasales Natural-Killer-/T-Zellen-Lymphom, Hodenkrebs (Seminom), Schilddrüsenkarzinom, malignes Melanom, cystisches Adenokarzinom, Angiosarkom, großzelliganaplastisches Lymphom, Endometriumkarzinom oder Prostatakarzinom handelt.

## Revendications

1. Composé représenté par la formule (I) : 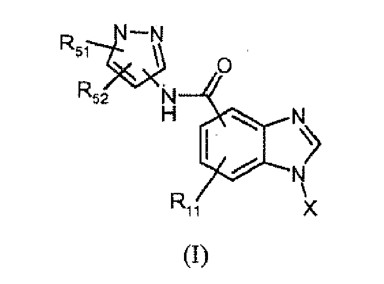 ou sel ou N-oxyde pharmaceutiquement acceptable de celui-ci, dans laquelle
R₁₁ est F, Cl, C₀₋₈alkyle, C₀₋₈alcoxy ou -N(C₀₋₈alkyl) (C₀₋₈alkyle) ;
R₅₁, est phényle, éventuellement substitué par 1-5 substituants indépendants halogène, -NR₃₄R₃₅, -NR₃₄COR₃₅, -NR₃₄C(O)OR₃₅, -NR₃₄SO₂R₃₅, -OR₃₄, -SR₃₄, -SO₂R₃₄, -SO₂NR₃₄R₃₅, -C(O)OR₃₄, -CO₂H, -CONR₃₄R₃₅, C₀₋₈alkyle, C₂₋₈alcényle, C₂₋₈alcynyle, CN, CF₃, NO₂, oxo, carbocycliques ou hétérocyclyle ;
R₅₂ est halogène, CF₃, -CN, C₀₋₈alkyle, C₀₋₈alcoxy, -COOH ou -N(C₀₋₈alkyl) (C₀₋₈alkyle) ;
X est un groupement carbocyclique ou hétérocyclyle, éventuellement substitué par 1-4 substituants indépendants F, Cl, CF₃, -CN, C₀₋₈alkyle, C₀₋₈alcoxy, -COOH ou -N(C₀₋₈alkyl)(C₀₋₈alkyle) ;
R₃₄ et R₃₅ sont indépendamment C₀₋₈alkyle éventuellement substitué par un substituant hétérocyclyle ou OH ; -C₀₋₈alkyl-C₃₋₈cycloalkyle, CF₃, -C₀₋₈alkyl-O-C₀₋₈alkyle, -C₀₋₈alkyl-N(C₀₋₈alkyl)(C₀₋₈alkyle), -C₀₋₈alkyl-S(O)₀₋₂-C₀₋₈alkyle ; ou hétérocyclyle éventuellement substitué par C₀₋₈alkyle, un substituant carbocyclique ou carbocyclique substitué.

2. Composé selon la revendication 1, ou sel ou N-oxyde pharmaceutiquement acceptable de celui-ci, **caractérisé en ce que** X est un groupement carbocyclique éventuellement substitué.

3. Composé selon la revendication 2, ou sel ou N-oxyde pharmaceutiquement acceptable de celui-ci, **caractérisé en ce que** X est phényle éventuellement substitué.

4. Composé selon la revendication 1, constitué de
1-(3-fluorophényl)-*N*-[3(5)-phénylpyrazol-5(3)-yl]-1*H*-benzimidazole-5-carboxamide ;
1-(3-fluorophényl)-*N*-[3(5)-(4-chlorophényl)pyrazol-5(3)-yl]-1*H*-benzimidazole-5-carboxamide ;
1-(3-fluorophényl)-*N*-[3(5)-*p*-tolylpyrazol-5(3)-yl]-1*H*-benzimidazole-5-carboxamide ;
1-(3-fluorophényl)-*N*-(1-methyl-3-phénylpyrazol-5-yl)-1*H*-benzimidazole-5-carboxamide ;
1-(4-méthoxyphényl)-*N*-(3(5)-phénylpyrazol-5(3)-yl)-1*H*-benzimidazole-5-carboxamide ;
1-[3-méthoxy-5-(trifluorométhyl)phényl]-*N*-(3(5)-phénylpyrazol-5(3)-yl)-1*H*-benzimidazole-5-carboxamide ;
1-(3,5-diméthoxyphényl]-*N*-(3(5)-phénylpyrazol-5(3)-yl)-1*H*-benzimidazole-5-carboxamide ;
1-(3-isopropoxyphényl)-*N*-(3(5)-phénylpyrazol-5(3)-yl)-1*H*-benzimidazole-5-carboxamide ; ou
1-(4-bromophényl)-*N*-(3(5)-phénylpyrazol-5(3)-yl)-1*H*-benzimidazole-5-carboxamide ;
ou sel ou *N*-oxyde pharmaceutiquement acceptable de celui-ci.

5. Composition comprenant un composé selon l'une quelconque des revendications 1 à 4, ou un sel ou un N-oxyde pharmaceutiquement acceptable de celui-ci, et un support pharmaceutiquement acceptable.

6. Composition comprenant un composé selon l'une quelconque des revendications 1 à 4, ou un sel ou un N-oxyde pharmaceutiquement acceptable de celui-ci, et un agent anti-néoplasique, anti-tumoral, anti-angiogénique ou chimiothérapeutique.

7. Composition comprenant un composé selon l'une quelconque des revendications 1 à 4, ou un sel ou un N-oxyde pharmaceutiquement acceptable de celui-ci, et un agent thérapeutique anticancéreux cytotoxique.

8. Composition comprenant un composé selon l'une quelconque des revendications 1 à 4, ou un sel ou un N-oxyde pharmaceutiquement acceptable de celui-ci, et un agent thérapeutique anticancéreux inhibant l'angiogénèse.

9. Utilisation d'un composé selon l'une quelconque des revendications 1 à 4, ou d'un sel ou d'un N-oxyde pharmaceutiquement acceptable de celui-ci, dans la fabrication d'un médicament destiné au traitement d'un trouble hyperprolifératif.

10. Utilisation selon la revendication 9, **caractérisée en ce que** le médicament est adapté à l'administration avec un agent anti-néoplasique, anti-tumoral, anti-angiogénique ou chimiothérapeutique.

11. Utilisation selon la revendication 9, **caractérisée en ce que** le trouble hyperprolifératif est le cancer du sein, le cancer de la tête, le cancer du cou, le cancer gastro-intestinal, les leucémies, le cancer de l'ovaire, des bronches, du poumon ou du pancréas, ou le cancer du poumon à petites cellules ou du colon.

12. Utilisation selon la revendication 9, **caractérisée en ce que** le trouble hyperprolifératif est le lymphome sinonasal à cellules T/tueuses naturelles, le cancer des testicules (séminome), le carcinome de la thyroïde, le mélanome malin, le carcinome adénoïde kystique, l'angiosarcome, le lymphome anaplasique à grandes cellules, le carcinome de l'endomètre, ou le carcinome de la prostate.

13. Composé selon l'une quelconque des revendications 1 à 4, ou sel ou N-oxyde pharmaceutiquement acceptable de celui-ci, destiné à être utilisé dans le traitement d'un trouble hyperprolifératif.

14. Composé selon la revendication 13, **caractérisé en ce que** le composé est destiné à l'administration avec un agent anti-néoplasique, anti-tumoral, anti-angiogénique ou chimiothérapeutique.

15. Composé selon la revendication 13, **caractérisé en ce que** le trouble hyperprolifératif est le cancer du sein, le cancer de la tête, le cancer du cou, le cancer gastro-intestinal, les leucémies, le cancer de l'ovaire, des bronches, du poumon ou du pancréas, ou le cancer du poumon à petites cellules ou du colon.

16. Composé selon la revendication 13, **caractérisé en ce que** le trouble hyperprolifératif est le lymphome sinonasal à cellules T/tueuses naturelles, le cancer des testicules (séminome), le carcinome de la thyroïde, le mélanome malin, le carcinome adénoïde kystique, l'angiosarcome, le lymphome anaplasique à grandes cellules, le carcinome de l'endomètre, ou le carcinome de la prostate.
